(19) Europäisches Patentamt
European Patent Office
Office européen des brevets

(11) Publication number: **0 241 975 B1**

(12) # EUROPEAN PATENT SPECIFICATION

(45) Date of publication of patent specification: **13.03.91**

(51) Int. Cl.$^5$: **C07C 381/02**, C08K 5/41, C08L 21/00

(21) Application number: **87200597.0**

(22) Date of filing: **31.03.87**

(54) **Process for the preparation of organic thiosulphates.**

(30) Priority: **02.04.86 GB 8608052**

(43) Date of publication of application:
**21.10.87 Bulletin 87/43**

(45) Publication of the grant of the patent:
**13.03.91 Bulletin 91/11**

(84) Designated Contracting States:
**AT BE CH DE ES FR GB GR IT LI LU NL SE**

(56) References cited:
**EP-A- 0 070 143**

(73) Proprietor: **SHELL INTERNATIONALE RESEARCH MAATSCHAPPIJ B.V.**
**Carel van Bylandtlaan 30**
**NL-2596 HR Den Haag(NL)**

(72) Inventor: **Bassett, Jean-Marie**
**Shell Centre**
**London SEI 7NA(GB)**

(74) Representative: **Aalbers, Onno et al**
**P.O. Box 302**
**NL-2501 CH The Hague(NL)**

**EP 0 241 975 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

## Description

This invention relates to a process for the preparation of organic thiosulphates useful as stabilisers or as metal-to-rubber adhesion promoters in sulphur-vulcanisable rubber compositions.

European Patent Application Publication No. 70 143 (EP-A-70 143) discloses a class of organic thiosulphates and thiosulphonates useful as stabilising agents for rubber vulcanisates. These stabilising agents are materials containing two or more groups of the formula

$-S-SO_2R$

where R represents (a) a radical -OM where M is a monovalent metal, the equivalent of a multivalent metal, a monovalent ion derived by the addition of a proton to a nitrogenous base or the equivalent of a multivalent ion derived by the addition of two or more protons to a nitrogenous base, or (b) an organic radical selected from aliphatic, cycloaliphatic, aromatic and heterocyclic radicals, and radicals which are combinations of any two or more such radicals, the groups of the aforesaid formula being linked by an organic bridging group or attached to an organic polymer chain. The groups are thus thiosulphate groups $-S-SO_2OM$ or thiosulphonate groups $-S-SO_2R$ in which R is an aforesaid organic radical.

A preferred sub-class of the above materials has the formula

$RO_2S-S-X'-S-SO_2R$

wherein R is as defined above and $X'$ can be, for example, a straight- or branched-chain alkylene or alkenylene group, preferably one containing 2 or from 5 to 40 carbon atoms, and more preferably one containing 5 to 16 carbon atoms. Examples of such groups are ethylene, pentamethylene, hexamethylene, octamethylene, nonamethylene, decamethylene, dodecamethylene, 3-methyl-1,5-pentylene and 1,6-hex-2-enylene.

When M in the above formula of the stabiliser material represents a monovalent metal, this can be for instance an alkali metal, for example sodium, lithium or potassium. Sodium is the preferred alkali metal. M can alternatively represent the equivalent of a multivalent metal, for instance magnesium, calcium, barium, zinc, nickel, cobalt or aluminium.

Where M represents a monovalent ion formed by the addition of a proton to a nitrogenous base, the nitrogenous base can be ammonia or a simple primary, secondary or tertiary amine

$R^2NH_2$, $R^2R^3NH$ or $R^2R^3R^4N$

where each of $R^2$, $R^3$ and $R^4$ independently represents an alkyl group, for example a $C_{1-20}$ alkyl group, a $C_{5-9}$ cycloalkyl or alkylcycloalkyl group, for example cyclohexyl or methylcyclohexyl, a benzyl group, a phenyl group or a substituted phenyl group, for example a tolyl or chlorophenyl group, provided that not more than one of $R^2$, $R^3$ and $R^4$ is a phenyl or substituted phenyl group.

Preferred amines are those that are relatively weakly basic. These include amines where weak basicity is a result of steric hindrance around the nitrogen atom due, for example, to the presence of a tert-alkyl group, for instance a tert-alkyl group having from 4 to 12 carbon atoms, such as tert-butyl, tert-amyl or 1,1,3,3-tetramethylbutyl. Examples of such amines are the secondary amines $R^2R^3NH$ where one of $R^2$ and $R^3$ is a tert-alkyl group and the other is a benzyl group or a cyclohexyl or alkylcyclohexyl group. Alternatively both $R^2$ and $R^3$ can be tert-alkyl groups. Further examples are tertiary amines where $R^2$ is a tert alkyl group and $R^3$ and $R^4$ are benzyl groups.

Other suitable weakly basic amines are the primary amines $R^2NH_2$ where $R^2$ is a phenyl or substituted phenyl group, and the secondary amines $R^2R^3NH$ where $R^2$ is a phenyl or substituted phenyl group and $R^3$ is a $C_{1-20}$ alkyl group, preferably a $C_{1-12}$ alkyl group. Examples of such amines are aniline, the toluidines, N-methylaniline, N-butylaniline and N-isohexylaniline. A special class of such secondary amines comprises those where $R^2$ represents a secondary alkyl group, preferably a $C_{3-12}$ secondary alkyl group, or a cyclohexyl group, and $R^3$ represents a 4-phenylaminophenyl group. These amines include compounds such as N-isopropyl-N'-phenyl-p-phenylenediamine, N-sec-butyl-N'-phenyl-p-phenylenediamine, N-1,3-dimethylbutyl-N'-phenyl-p-phenylenediamine, N-1,4-dimethylpentyl-N'-phenyl-p-phenylenediamine and N-cyclohexyl-N'-phenyl-p-phenylenediamine. Such amines function as mono-acid bases despite the presence of the second nitrogen atom in the 4-phenylaminophenyl group, because this second nitrogen atom has virtually no basicity.

Other examples of nitrogenous bases which form thiosulphate salts are guanidine and substituted guanidines, for example those of the formula

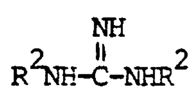

and substituted isothioureas, for example those of the formula

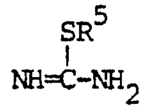

where each $R^2$ independently represents hydrogen, an alkyl group, for example a $C_{1-20}$ alkyl group, a $C_{5-9}$ cycloalkyl or alkylcycloalkyl group, a benzyl group, a phenyl group or a substituted phenyl group; for instance a tolyl group, and $R^5$ represents a $C_{1-20}$ alkyl group, a $C_{5-9}$ cycloalkyl or alkylcycloalkyl group or a benzyl group. Specific examples of substituted guanidines are diphenylguanidine and di-o-tolylguanidine; specific examples of substituted isothioureas are S-ethylisothiourea and S-benzylisothiourea.

Where M represents an equivalent of a multivalent cation formed by the addition of two or more protons to a nitrogenous base, the bases from which such ions can be derived include alkylene diamines, N,N′-disubstituted alkylene diamines, phenylenediamines and N,N′-disubstituted phenylenediamines of the formula

$R^2NH\text{-}A\text{-}NHR^2$

where A represents an alkylene radical $-(CH_2)_c-$ where c has a value of from 2 to 20, preferably from 2 to 12, and which may be straight chain or branched, or a phenylene, for example a meta-or para-phenylene radical, and each $R^2$ independently represents an alkyl group, for example a $C_{1-20}$ alkyl group, a $C_{5-9}$ cycloalkyl or alkylcycloalkyl group, a benzyl group, a phenyl group or substituted phenyl group, provided that neither $R^2$ is a phenyl or substituted phenyl group when A is a phenylene radical.

In preferred amines where A represents an alkylene radical, $R^2$ is a tert-alkyl group, for example tert-butyl, t-amyl or 1,1,3,3-tetramethylbutyl, or a phenyl group. Examples of such amines are N,N′-diphenylethylene diamine,
N,N′-di-tert-butyl-1,4-tetramethylene diamine and
N,N′-bis(1,1,3,3-tetramethylbutyl)-1,6-hexamethylene diamine.

In preferred amines where A represents a phenylene radical, $R^2$ is a secondary alkyl group, preferably a $C_{3-12}$ secondary alkyl group or a cyclohexyl group. Examples of such amines are
N,N′-di-sec-butyl-p-phenylenediamine,
N,N′-bis(1,3-dimethylbutyl)-p-phenylenediamine,
N,N′-bis(1,4-dimethylpentyl)-p-phenylenediamine,
N,N′-bis(1-ethyl-3-methylpentyl)-p-phenylenediamine,
N.N′-bis(1-methylheptyl)-p-phenylenediamine and
N,N′-dicyclohexyl-p-phenylenediamine.

Possible bases also include polyalkylene polyamines of the formula

$R^2NH\text{-}(A'\text{-}NH)_n\text{-}A'NHR^2$

where A′ represents an alkylene radical of from 2 to 8 carbon atoms, n has a value of from 1 to 5, and each $R^2$ independently represents a $C_{1-20}$ alkyl group, a $C_{5-9}$ cycloalkyl or alkylcycloalkyl group, a benzyl group, a phenyl group or a substituted phenyl group.

In other instances, the nitrogen of the nitrogenous base is part of a heterocyclic ring. The base can be monocyclic, for example pyridine, or a compound in which the nitrogen-containing heterocyclic ring is fused to another ring, as for example quinoline. Moreover, the heterocyclic ring can be saturated, as for example in morpholine or piperidine, or it may contain one or more double bonds, as for example in pyrroline or 1,2-dihydroquinoline.

Of the compounds where M represents such a base, those preferred for use as vulcanisate stabilisers are compounds where M represents a 1,2-dihydroquinolinium ion, which may optionally have ring substituents. Examples of such ions are
2,2,4-trimethyl-1,2-dihydroquinolinium,
2,2,4-trimethyl-6-($C_{1-12}$alkoxy)-1,2-dihydroquinolinium, for instance 2,2,4-trimethyl-6-ethoxy-1,2-dihydroquinolinium,
2,2,4-trimethyl-6-($C_{1-18}$alkyl)-1,2-dihydroquinolinium, for instance 2,2,4-trimethyl-6-dodecyl-1,2-dihydroquinolinium, and
2,4-diethyl-2-methyl-1,2-dihydroquinolinium.

Other classes of bases which form divalent cations by the addition of two protons are represented by the general formulae

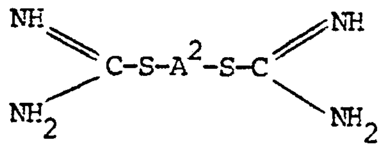

and

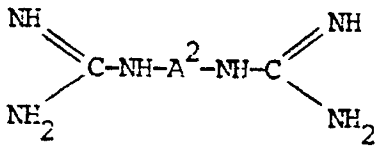

where $A^2$ represents a radical $-(CH_2)_c-$, where c is an integer from 2 to 20, preferably from 3 to 12, and the radical $-(CH_2)_c-$ can be either straight chain or branched or a $C_{2-20}$ alkenylene or alkadienylene radical, for example a but-2-enylene or octa-2,6-dienylene radical. These bases form bis(isothiouronium) and bis-(guanidinium) ions respectively.

One process described in EP-A-70143 for the preparation of the above sub-class of materials wherein M represents an alkali metal is reaction of a compound of formula $X'(Hal)_2$, where Hal represents halogen, with an alkali metal thiosulphate, preferably sodium thiosulphate, e.g. in water or aqueous alcoholic medium under reflux. Particularly when Hal is chlorine, reaction tends to be slow and the reaction may be effected under pressure, in an autoclave at temperatures in the range 100 to 150°C, preferably 120 to 140°C. If desired ethylene glycol or diethylene glycol may be used as solvent, and temperatures over 100°C can be achieved without use of elevated pressures.

Alkali metal salts of organic thiosulphates prepared and isolated using glycol solvents may contain glycol relatively firmly bound in the crystal. So far as the use of the thiosulphate as a rubber stabiliser is concerned, the presence of small amounts of glycol has no adverse effect, but if desired, the glycol can be removed by recrystallisation from a non-glycol solvent.

Stabilisers of EP-A-70 143 where M represents potassium can be made by using potassium thiosulphate as the halogen-displacing reactant. For the preparation of compounds having other values of M, however, it is in many instances most convenient to prepare the sodium salt as an intermediate from which the sodium is then displaced by the required other cation.

Where the required product is water-soluble, such a displacement can be effected using a cation-exchange resin which carries the required other cation. For example, introduction of a solution of the sodium salt of the organic thiosulphate into a column of cation-exchange resin in which the exchangeable ions are nickel produces as a percolate a solution of the nickel salt of the organic thiosulphate. By essentially the same method, using a cation-exchange resin carrying the cations required in the product, magnesium, calcium, zinc, cobalt and guanidinium salts of the organic thiosulphates can be prepared. The salts in solid form, often containing water of crystallisation, can be obtained by evaporation of the percolates.

The barium salts of the organic thiosulphates are less soluble in water than the alkali metal and certain other metal salts, and crystallise on cooling a solution obtained by mixing hot, concentrated solutions of barium chloride and the organic thiosulphate sodium salt. The barium salts are useful as intermediates in the preparation of other metal salts by double decomposition. Addition of an aqueous solution of the sulphate of the other metal to an aqueous solution of the barium salt (which can be obtained using a sufficient volume of water) results in the precipitation of barium sulphate. This is removed by filtration, giving a filtrate which on evaporation yields the desired metal salt of the organic thiosulphates. Ammonium and certain substituted ammonium salts can also be prepared by this procedure.

Double decomposition procedures using as reactants the alkali metal salts, especially the sodium salts, of the organic thiosulphates and the salts of nitrogenous bases with strong mineral acids, for example hydrochlorides, hydrobromides or sulphates, can be used to prepared the stabiliser materials where M represents a monovalent ion formed by the addition of a proton, or the equivalent of a multivalent ion formed by the addition of two or more protons, to an organic nitrogenous base. The by-product is an alkali metal salt of a strong mineral acid, for example sodium chloride or sodium sulphate, and its separation from the required product is usually straightforward by virtue of their differing solubilities in selected solvents. For instance, the sodium salts of the organic thiosulphates dissolve to a limited extent in warm methanol, as do the sulphates of certain amines, whereas sodium sulphate is virtually insoluble in methanol. On mixing a warm methanolic solution of a sodium salt of an organic thiosulphate with a warm methanolic solution of an

amine sulphate, sodium sulphate is precipitated, and can be separated by filtration from the amine salt of the organic thiosulphate which remains in solution. The amine salt itself can be obtained by evaporation of the solvent from the filtrate. This method can be used to prepare salts of amines $R^2R^3NH$ where $R^2$ represents a secondary alkyl group or a cyclohexyl group, and $R^3$ represents a 4-phenylamino group, as well as salts were the cation is an optionally-substituted 1,2-dihydroquinolinium ion.

In other instances, the amine salt of the organic thiosulphate is relatively insoluble in water or aqueous alcohol, and crystallises from the solution obtained by mixing an aqueous or aqueous alcoholic solution of the amine hydrochloride with an aqueous solution of the sodium salt of the organic thiosulphate. N-tert-alkyl-N-benzyl-ammonium, diphenylguanidinium and certain isothiouronium salts can be prepared by this method.

An alternative process outlined in EP-A-70 143 for the preparation of the above sub-class of materials comprises reaction of the dithiol HS-X′-SH with a compound of formula $RSO_2Cl$ in the presence of a base.

EP-A-109 955 discloses the use of materials disclosed in EP-A-70 143 as stabilising agents as adhesion promoters to promote the bonding of metal-to-rubber, e.g. bonding between rubber and brass-coated steel, in sulphur-vulcanisable rubber compositions.

EP-A-160 634 discloses compounds corresponding to the materials disclosed in EP-A-70 143 which have, in place of the moiety M therein defined, a complex cation of (divalent) nickel or cobalt with an amine, and their use as adhesion promoters to promote the bonding of metal-to-rubber in sulphur-vulcanisable rubber compositions. The compounds are formed by mixing in solution a source of nickel or cobalt ions, an alkali metal salt of the organic thiosulphate and an amine. Conveniently a solution containing the nickel or cobalt ions (e.g. as sulphate, chloride or acetate salt) is added to a solution of the other reactants.

Amines known to form complex cations with divalent nickel or cobalt include those of the general formulae $H_2N\text{-}R\text{-}NH_2$ where R represents a straight or branched chain group $CnH_{2n}$, n having a value of from 2 to 8, generally from 2 to 6, with the amine groups being separated by a sequence of no more than 3 carbon atoms. Examples of such amines are 1,2-diaminoethane, 1,3-diaminopropane, 1,2-diamino-2-methylpropane, 1,3-diamino-2,2-dimethylpropane, 2,2-diaminobutane, and 2,3-diamino-2,3-dimethylbutane.

Also known as complex-forming amines are those having the above formula in which R represents a 1-phenylethylene or 1,2-diphenylethylene radical, or a radical of the formula $-CH_2CH_2NHCH_2CH_2-$. A further instance of an amine known to form a complex with nickel or cobalt is 2,2′-bipyridyl.

In other instances, complexes have been obtained from amines which were not known to form well-defined cationic complexes with cobalt or nickel. These amines include N-benzyl-N-(tert-alkyl)amines, where the tert-alkyl group has the formula $-C(CH_3)_2\text{-}CH_2R^1$, where $R^1$ represents H or a straight or branched-chain alkyl group of from 1 to 8 carbon atoms. Specific examples of such amines are N-benzyl-N-t-butylamine and N-benzyl-N-t-octylamine (i.e. N-benzyl-N-(1,1,3,3-tetramethylbutyl)amine).

Other amines which form complexes are poly(ethylene imines).

The above-described prior art processes for the preparation of the organic thiosulphates have the prime disadvantage that the step wherein the organic thiosulphate ion is formed is accompanied by formation of undesired salts, e.g. sodium chloride, which can give separation problems. Where glycols are used as solvents, glycol may remain relatively firmly bound in the organic thiosulphate.

There has now been discovered an advantageous process for the preparation of certain organic thiosulphates which avoids co-formation of inorganic salts in formation of the organic thiosulphate ion, and wherein use of glycols as solvent is avoidable.

According to the present invention there is provided a process for the preparation of a compound of general formula

$M\text{-}O\text{-}O_2S\text{-}S\text{-}X\text{-}S\text{-}SO_2\text{-}O\text{-}M$(I)

where X represents an alkylene radical, and M represents a monovalent metal, the equivalent of a multivalent metal, a monovalent ion derived by the addition of a proton to a nitrogenous base, or the equivalent of a multivalent ion derived by the addition of two or more protons to a nitrogenous base or M represents the equivalent of a complex cation of nickel or cobalt with an amine, which process comprises reacting a dithiol of general formula

H-S-X-S-H(II)

where X is as defined above with a sulphur trioxide-base complex of formula

$SO_3.A$(III)

where A is a nitrogenous base selected from optionally substituted pyridine, optionally substituted quinoline and tertiary amines of formula $NR^1R^2R^3$ wherein $R^1$, $R^2$ and $R^3$ are independently selected from $C_{1\text{-}20}$ alkyl, preferably $C_{1\text{-}10}$ alkyl, phenyl and benzyl groups to produce a compound of formula I wherein M represents a monovalent ion $AH^+$, optionally followed by reaction with an alkali metal hydroxide to produce a compound of formula I wherein M represents an alkali metal, optionally followed by conversion of the

compound of formula I wherein M represents an alkali metal into another compound of formula I wherein M is as defined above.

Conversion of a compound of formula I wherein M represents an alkali metal into another compound of formula I wherein M is as defined above may be achieved as described generally above in relation to EP-A-70 143 and EP-A-160 634, and the various moieties M may be as defined therein, or as described specifically in EP-A-70 143 or EP-A-160 634.

In formula I, X preferably represents a $C_{5-20}$ alkylene group, which may be linear (i.e. a polymethylene group) or branched. It is further preferred for X to represent a group $-(CH_2)_{n-1}-CHR-$ where n is 5 to 20 and R is a hydrogen atom or a $C_{1-6}$ alkyl group, provided that R contains not more than (20-n) carbon atoms. R is preferably a hydrogen atom. Advantageously X represents a $C_{5-10}$ alkylene group, preferably a hexamethylene group.

The nitrogenous base A preferably represents pyridine optionally substituted by 1 to 3 methyl groups, e.g. picoline (3-methyl pyridine), quinoline or a tertiary amine $NR^1R^2R^3$ wherein $R^1$, $R^2$ and $R^3$ are independently selected from $C_{1-6}$ alkyl groups, e.g. tri-n-propylamine or tri-n-butylamine, preferably a tri-($C_{1-2}$ alkyl)amine, e.g. trimethylamine or triethylamine. Pyridine and trimethylamine have been found to be very effective.

Reaction of the dithiol of formula II and the sulphur trioxide base complex of formula III may very conveniently be effected in the presence of a halogenated hydrocarbon as solvent. Liquid chlorinated alkanes, especially di- or trichloro-$C_{1-4}$ alkanes, are very suitable.

Examples of such chlorinated alkanes include methylene chloride (b.p. 39 to 40 °C), 1,2-dichloroethane (b.p. 83 °C to 84 °C), 1,1,2-trichloroethane (b.p. 113 °C to 114 °C), 1,1,1-trichloroethane (b.p. 74 °C to 75 °C), 1,1-dichloropropane (b.p. 87 °C), 1,2-dichloropropane (b.p. 95 °C to 96 °C) and 1,3-dichloropropane (b.p. 125 °C). Other such halogenated hydrocarbons will be immediately apparent to those skilled in the art. Methylene chloride and 1,2-dichloroethane have been found to be very suitable.

Reaction of the dithiol of formula II and the sulphur trioxide-base complex of formula III may conveniently be effected at a temperature in the range 20 °C to reflux temperature. Preferably the temperature is at least 30 °C, advantageously at least 35 °C. The most preferred temperature range is 45 °C to reflux temperature.

The sulphur-trioxide-base complex may conveniently be prepared by reacting stoichiometric amounts of nitrogenous base A and sulphur trioxide in the presence of an inert solvent, e.g. 1,2-dichloroethane, with cooling, e.g. at 0 °C.

The compound of formula I wherein M represents a monovalent ion $AH^+$ is preferably reacted with an alkali metal hydroxide to produce a compound of formula I wherein M represents an alkali metal. The alkali metal hydroxide is preferably sodium hydroxide. Sodium salts such as sodium hexamethylene bisthiosulphate are particularly desired compounds of formula I.

If desired the reaction with the alkali metal hydroxide can be effected without first isolating the compound of formula I wherein M represents the monovalent ion $AH^+$. Those skilled in the art will appreciate that a stoichiometric quantity of alkali metal hydroxide should desirably be employed to achieve complete conversion to the alkali metal salt and to avoid possible contamination of the end product. The reaction with the alkali metal hydroxide may conveniently be effected at ambient temperature. The base A may be removed from aqueous medium by evaporation together with water and subsequently separated for subsequent recycling (reaction with sulphur trioxide to form further sulphur trioxide-base complex). The resulting alkali metal salt of formula I may be precipitated from aqueous medium after the evaporation by addition of a water-miscible solvent such as acetone or isopropanol.

The invention further comprises compounds of formula I as defined above whenever prepared by the process of the invention, and to the use of those compounds as stabilisers or metal-to-rubber adhesion promoters in sulphur-vulcanisable rubber compositions. Suitable such compositions and applications thereof are described in EP-A-70 143, EP-A-109 955 and EP-A-160 634.

It has been found that dithiols of formula II may very conveniently be prepared from the corresponding, readily available, dienes. Where X represents a group $-(CH_2)_{n-1}-CHR-$, the corresponding diene has the formula $CH_2=CH-(CH_2)_{n-4}-CH=CHR$. Thus when R is a hydrogen atom, the diene is an $\alpha,\omega$-diene. The diene is reacted with thiolacetic acid in the presence of a free-radical initiator (e.g. t -butylperpivalate or 2,2′-azobisisobutyronitrile), preferably at a temperature in the range 60 °C to reflux temperature, and the resulting bisthiolacetate is hydrolysed in the presence of a base, preferably an alkali metal hydroxide (advantageously sodium hydroxide), preferably at a temperature in the range 80 °C to reflux temperature, followed by neutralisation to yield the desired dithiol of formula II.

The invention will be further understood from the following illustrative Examples.

EXAMPLE 1

Preparation of sodium hexamethylene bisthiosulphate

(a) Preparation of 1,6-hexamethylene bisthiolacetate

To thiolacetic acid (61.4g, 0.8mol) was added 1,5-hexadiene (28.6g, 0.35mol) and t -butylperpivalate (0.6g), and the mixture was heated at 70 to 80°C for 5 hours. Evaporation of excess thiolacetic acid (8.4g) and monoester (3.3g) under reduced pressure ($0.1 \times 10^5$Pa) left 1,6-hexamethylene bisthiolacetate (78.3g, 0.33mol, 94%) as product.

(b) Preparation of 1,6-hexamethylene bisthiol

The product of step (a) (78.3g) was heated at 100°C with 4 molar aqueous sodium hydroxide (343ml, 1.4mol NaOH) in the presence of methyl tri-n-octyl ammonium chloride (0.8g) (as phase-transfer catalyst) for 1 hour. The mixture was allowed to cool and was neutralised to pH 8 by addition of concentrated (36%w/v) aqueous hydrochloric acid (67ml, 0.66mol HCl). Extraction with hexane (200ml) followed by evaporation of the hexane gave 1,6-hexamethylene bisthiol (45g, 0.3mol, 90%), bp 89 to 91°C at 500 Pa, as product.

(c) Preparation of di-pyridinium hexamethylene bisthiosulphate

The product of step (b) (45g) was dissolved in methylene chloride (300ml) and sulphur trioxide-pyridine (96g, 0.6mol). The resulting mixture was heated at 45 to 50°C (reflux) for 1½ hours, and then allowed to cool. White crystals which separated out were filtered off to afford di-pyridinium hexamethylene bisthiosulphate (14.2g, 0.3mol) as product.
1H NMR, δ (ppm), 1.4(m,4H), 1.7(m,4H), 3.1(t,4H), 8.11(t,2H), 8.75(m,3H).

(d) Preparation of disodium hexamethylene bisthiosulphate

The product of step (c) (142g) was dissolved in a solution of sodium hydroxide (24g, 0.6mol) in water (160ml). The resulting solution was concentrated by evaporation of the pyridine and 60ml water, and to the resulting slurry was added acetone (150ml). The white crystals which separated out were filtered off to afford disodium hexamethylene bisthiosulphate dihydrate (109g, 0.28mol, 93%) as product. (1R absorption bands were identical with those given in EP-A-70143, i.e. 3,555 to 3,455cm $^{-1}$- water of crystallisation; 2,920, 2,855 and 1,465cm$^{-1}$- $-CH_2-$; 1,220, 1,050 and 645cm$^{-1}$ - $-SSO_3$, in KBr wafer).

EXAMPLE 2

The procedure of Example 1 was followed except that in steps (c) and (d) the white crystals of di-pyridinium hexamethylene bisthiosulphate were not filtered off and isolated. Instead, the solution of sodium hydroxide (24g, 0.6mol) in water (160ml) was added directly to the bisthiosulphate/pyridine/methylene chloride mixture, the organic phase (methylene chloride and some pyridine) was separated off and the aqueous phase was concentrated by evaporation of the remaining pyridine and 60ml water. Further processing was as in Example 1(d), with identical yield of disodium hexamethylene bisthiosulphate dihydrate being obtained as product.

EXAMPLE 3

The procedure of Example 2 was followed except that in place of the sulphur trioxide-pyridine, there was employed sulphur trioxide-trimethylamine (83.4g, 0.6mol), and in place of methylene chloride as

solvent there was employed 1,2-dichloroethane. Reaction at 85°C (reflux) for 2½ hours gave quantitative conversion to the di-trimethylammonium hexamethylene bisthiosulphate (no starting material remaining) and the further treatment with sodium hydroxide resulted in identical yield of disodium hexamethylene bisthiosulphate dihydrate being obtained as in Example 1.

## Claims

1. A process for the preparation of a compound of general formula
$M-O-O_2S-S-X-S-SO_2-O-M$(I)
where X represents an alkylene radical, and M represents a monovalent metal, the equivalent of a multivalent metal, a monovalent ion derived by the addition of a proton to a nitrogenous base, or the equivalent of a multivalent ion derived by the addition of two or more protons to a nitrogenous base or M represents the equivalent of a complex cation of nickel or cobalt with an amine, which process comprises reacting a dithiol of general formula
H-S-X-S-H(II)
where X is as defined above with a sulphur trioxide-base complex of formula
$SO_3.A$(III)
where A is a nitrogenous base selected from optionally substituted pyridine, optionally substituted quinoline and tertiary amines of formula $NR^1R^2R^3$ wherein $R^1$, $R^2$ and $R^3$ are independently selected from $C_{1-20}$ alkyl, phenyl and benzyl groups to produce a compound of formula I wherein M represents a monovalent ion $AH^+$, optionally followed by reaction with an alkali metal hydroxide to produce a compound of formula I wherein M represents an alkali metal, optionally followed by conversion of the compound of formula I wherein M represents an alkali metal into another compound of formula I wherein M is as defined above.

2. A process according to Claim 1 wherein X represents a $C_{5-20}$ alkylene group.

3. A process according to Claim 1 or 2 wherein X represents a group $-(CH_2)_{n-1}-CHR-$ where n is 5 to 20 and R is a hydrogen atom or a $C_{1-6}$ alkyl group, provided that R contains not more than (20-n) carbon atoms.

4. A process according to any of Claims 1 to 3 wherein X represents a $C_{5-10}$ alkylene group.

5. A process according to any of Claims 1 to 4 wherein X represents a hexamethylene group.

6. A process according to any of Claims 1 to 5 wherein A represents pyridine optionally substituted by 1 to 3 methyl groups, quinoline or a tertiary amine $NR^1R^2R^3$ wherein $R^1$, $R^2$ and $R^3$ are independently selected from $C_{1-6}$ alkyl groups.

7. A process according to Claim 6 wherein A represents pyridine or trimethylamine.

8. A process according to any of Claims 1 to 7 wherein reaction of the dithiol of formula II and the sulphur trioxide-base complex of formula III is effected in the presence of a halogenated hydrocarbon as solvent.

9. A process according to Claim 8 wherein the halogenated hydrocarbon is a di- or trichloro-$C_{1-4}$ alkane.

10. A process according to Claim 9 wherein the halogenated hydrocarbon is methylene chloride or 1,2-dichloroethane.

11. A process according to any of Claims 1 to 10 wherein reaction of the dithiol of formula II and the sulphur trioxide-base complex of formula III is effected at a temperature in the range 20°C to reflux temperature.

12. A process according to Claim 11 wherein the temperature is at least 35°C.

13. A process according to any of Claims 1 to 12 wherein the alkali metal hydroxide is sodium hydroxide.

**Revendications**

1. Un procédé de préparation d'un composé de formule générale
$M-O-O_2S-S-X-S-SO_2-O-M$ (I)
où X représente un radical alcoylène et M représente un métal monovalent, l'équivalent d'un métal polyvalent, un ion monovalent dérivé par l'addition d'un proton à une base azotée ou l'équivalent d'un ion polyvalent dérivé par l'addition de deux protons ou plus à une base azotée ou M représente l'équivalent d'un cation complexe de nickel ou de cobalt avec une amine, procédé qui comprend la réaction d'un dithiol de formule générale

$$R-S-X-S-H \qquad (II)$$

où X est tel que défini ci-dessus avec un complexe anhydride sulfurique-base de formule

$$SO_3.A \qquad (III)$$

où A est une base azotée choisie parmi la pyridine éventuellement substituée, la quinoléine éventuellement substituée et des amines tertiaires de formule $NR^11R^2R^3$ où $R^1$, $R^2$ et $R^3$ sont choisis indépendamment parmi des groupes alcoyle en $C_{1-20}$ phényle et benzyle pour produire un composé de formule I où M représente un ion $AH^+$, cela étant éventuellement suivi d'une réaction avec un hydroxyde de métal alcalin pour produire un composé de formule I où M représente un métal alcalin, cela étant éventuellement suivi de la conversion du composé de formule I où M représente un métal alcalin en un autre composé de formule I où M est tel que défini ci-dessus.

2. Un procédé selon la revendication 1, dans lequel X représente un groupe alcoylène en $C_{5-20}$.

3. Un procédé selon la revendication 1 ou 2, dans lequel X représente un groupe - $(CH_2)_{n-1-CHR}$, où n a une valeur de 2 à 5 et R est un atome d'hydrogène ou un groupe alcoyle en $C_{1-6}$, du moment que R ne contient pas plus de (20-n) atomes de carbone.

4. Un procédé selon l'une quelconque des revendications 1 à 3, dans lequel X représente un groupe alcoylène en $C_{5-10}$.

5. Un procédé selon l'une quelconque des revendications 1 à 4, dans lequel X représente un groupe hexaméthylène.

6. Un procédé selon l'une quelconque des revendications 1 à 5, dans lequel A représente la pyridine éventuellement substituée par 1 à 3 groupes méthyle, la quinoléine ou une amine tertiaire $NR^1R^2R^3$ où $R^1$, $R^2$ et R3 sont choisis chacun indépendamment parmi des groupes alcoyle en $C_{1-6}$.

7. Un procédé selon la revendication 6, dans lequel A représente la pyridine ou la triméthylamine.

8. Un procédé selon l'une quelconque des revendications 1 à 7, dans lequel la réaction du dithiol de formule II et du complexe anhydride sulfurique-base de formule III est conduite en présence d'un hydrocarbure halogéné comme solvant.

9. Un procédé selon la revendication 8, dans lequel l'hydrocarbure halogéné est un di- ou trichloroalcane en $C_{1-4}$.

10. Un procédé selon la revendication 9, dans lequel l'hydrocarbure halogéné est le chlorure de méthylène ou le l,2-dichloroéthane.

11. Un procédé selon l'une quelconque des revendications 1 à 10, dans lequel la réaction du dithiol de

formule II et du complexe anhydride sulfurique-base de formule III est conduite à une température comprise entre 20° C et la température de reflux.

12. Un procédé selon la revendication 11, dans lequel la température est d'au moins 35° C.

13. Un procédé selon l'une quelconque des revendications 1 à 12, dans lequel l'hydroxyde de métal alcalin est l'hydroxyde de sodium.

## Ansprüche

1. Verfahren zur Herstellung einer Verbindung der allgemeinen Formel
$M\text{-}O\text{-}O_2S\text{-}S\text{-}X\text{-}S\text{-}SO_2\text{-}O\text{-}M$ (I),
worin X einen Alkylenrest bedeutet und M ein einwertiges Metall, das Äquivalent eines mehrwertigen Metalles, ein einwertiges, aus der Addition eines Protons an eine stickstoffhältige Base abgeleitetes Ion oder das Äquivalent eines mehrwertigen, aus der Addition von zwei oder mehreren Protonen an eine stickenstoffhältige Base abgeleiteten Ions darstellt, oder M das Äquivalent eines Komplexkations von Nickel oder Kobalt mit einem Amin bedeutet, welches Verfahren ein Umsetzen eines Dithiols der allgemeinen Formel

$$H\text{-}S\text{-}X\text{-}S\text{-}H \qquad (II),$$

worin X wie vorstehend definiert ist, mit einem Schwefeltrioxid-Basen-Komplex der Fromel

$$SO_3.A \qquad (III),$$

worin A eine stickstoffhältige Base, ausgewählt unter gegebenenfalls substituiertem pyrdrin, gegebenenfalls substituiertem Chinoluin und tertiären Aminen der Formel $NR^1R^2R^3$, worin $R^1$, $R^2$ und $R^3$ und $R^3$ unabhängig voneinander unter $C_{1-20}$-Alkyl-, Phenyl-und Benzylgruppen ausgewählt sind, zur Ausbildung einer verbindung der Formel I, worin M ein einwertiges Ion $AH^+$ darstellt, umfaßt, woran sich gegebenenfalls eine Umsetzung mit einem Alkalimetallhydroxid zur Ausbildung einer Verbindung der Formel I, worin M ein Alkalimetall darstellt, anschließt, woran sich gegebenenfalls eine Umwandlung der Verbindung der Formel I, worin M ein Alkalimetall darstellt, in eine andere Verbindung der Formel I, worin M wie oben definiert ist, anschließt.

2. Verfahren nach Anspruch 1, worin X eine $C_{5-20}$-Alkylengruppe bedeutet.

3. Verfahren nach Anspruch 1 oder 2, worin X eine Gruppe $-(CH_2)_{n-1}$-CHR-bedeutet, worin n 5 bis 20 ist und R ein Wasserstoffatom oder eine $C_{1-6}$-Alkylgruppe darstellt, mit der Maßgabe, daß R nicht mehr als (20-n) Kohlenstoffatome enthält.

4. Verhfahren nach einem der Ansprüche 1 bis 3, worin X eine $C_{5-10}$-Alky-lengruppe darstellt.

5. Verfahren nach einem der Ansprüche 1 bis 4, worin X eine Hexamethylengruppe bedeutet.

6. Verfahren nach einem der Ansprüche 1 bis 5, worin A für pyridin, des gegebenenfalls durch 1 bis 3 methylgruppen substituiert ist, für Chinolin oder für ein tertiäres Amin $NR^1R^2R^3$ steht, worin $R^1$, $R^2$ und $R^3$ unabhängig voneinander unter $C_{1-6}$ -Alkylgruppen ausgewählt sind.

7. Verfahren nach Anspruch 6, worin A Pyridin oder Trimethylamin bedeute

8. Verfahren nach einem der Ansprüche 1 bis 7, worin die Reaktion des Dithiols der Formel II mit dem Schwefeltrioxid-Basen-Komplex der Formel III in Anwesenheit eines halogenierten Kohlenwasserstoffes als Lösungsmittel ausgeführt wird.

9. Verfahren nach Anspruch 8, worin der halogenierte Kohlenwasserstoff ein Di- oder Trichlor-$C_{1-4}$-alkan ist.

**10.** Verfahren nach Anspruch 9, worin der halogenierte Kohlenwasserstoff Methylenchlorid oder 1,2-Dichlorethan ist.

**11.** Verfahren nach einen der Ansprüche 1-10, worin die Umsetzung des Dithiols der Formel II mit dem Schwefeltrioxid-Basen-Komplex der Formel III bei einer Temperatur im Bereich von 20° C bis zur Rückflußtemperatur ausgeführt wird.

**12.** Verfahren nach Anspruch 11, worin die Temperatur wenigstens 35° C beträgt.

**13.** Verfahren nach einem der Ansprüche 1 bis 12, worin das Alkalimetallhydroxid Natriumhydroxid ist.